# EUROPEAN PATENT APPLICATION

(11) **EP 4 050 461 A1**
(43) Date of publication of application: **31.08.2022**
(21) Application number: 21159065.8
(22) Date of filing: 24.02.2021
(51) Int. Cl.: G06F 3/01, A61B 5/00, A61N 5/06

(54) **TREATMENT GUIDANCE**

(71) Applicant: Koninklijke Philips N.V., 5656 AG Eindhoven (NL)
(72) Inventor: Palero, Jonathan Alambra, 5656 AE Eindhoven (NL); Bourquin, Yannyk Parulian Julian, 5656 AE Eindhoven (NL); Damodaran, Mathivanan, 5656 AE Eindhoven (NL); Asselman, Michel Jozef Agnes, 5656 AE Eindhoven (NL)
(74) Representative: Philips Intellectual Property & Standards

(57) **Abstract**

According to an aspect, there is provided a treatment guidance system (100) for guiding a user, using a treatment applicator (104), during treatment of a treatment object on a surface of a body part of the user. The system comprises at least one image capture device (106) configured to capture an image of the surface of the body part; a localization unit (108) configured to determine a location of a treatment element of the treatment applicator relative to the body part; a display (110) configured to present information to the user; and a processor (112). The processor is configured to control the at least one image capture device to capture a plurality of images of the surface of the body part; determine, based on the captured images, a location of a treatment object in the captured images; generate, based on the captured images, a three-dimensional representation of the surface of the body part, the three-dimensional representation including an indication of the treatment object in a location corresponding to the determined location of the treatment object in the captured images, and an indication of the location of the treatment element as determined by the localization unit; and provide, for presentation on the display to the user, the three-dimensional representation of the surface of the body part, including the indication of the treatment object and the indication of the location of the treatment element.

## Description

### FIELD OF THE INVENTION

The invention relates to treatment guidance and, more particularly, to guiding a user during treatment of a treatment object of the surface of a body part of the user.

### BACKGROUND OF THE INVENTION

When a person performs a personal care activity, they may use a personal care device. Similarly, when a person performs treatment of part of their body, they may use a treatment device or applicator, or they may apply the treatment using their finger, for example. If the personal care activity or treatment is to be performed in respect of a part of the person's body that they cannot easily see directly (e.g. their face), then the person may use the device such as a mirror to aid the activity.

Even if a mirror is used when performing a personal care activity or a treatment activity, ensuring that the treatment is applied onto their body at the intended location can be difficult if, for example, the person's view of the treatment location on their body is obscured or blocked by their hand, the personal care device or the treatment applicator.

It would therefore be useful to have a system capable of providing guidance to the user, which at least partially addresses the above-identified problem.

### SUMMARY OF THE INVENTION

A person may be able to apply treatment to their body more accurately if, during the treatment, they are able to know and/or see the location of the treatment applicator relative to the part of their body to which the treatment is being applied. The inventors of the present disclosure have recognized that, a treatment activity can be improved for a person if the person is presented with a representation of the body part being treated which also indicate the location of the treatment applicator. That way, the presence of user's hand and/or the treatment applicator being used for the treatment will not block or obscure the user's view of the body part being treated.

According to a first specific aspect, there is provided a treatment guidance system for guiding a user, using a treatment applicator, during treatment of a treatment object on a surface of a body part of the user, the system comprising at least one image capture device configured to capture an image of the surface of the body part; a localization unit configured to determine a location of a treatment element of the treatment applicator relative to the body part; a display configured to present information to the user; and a processor. The processor is configured to control the at least one image capture device to capture a plurality of images of the surface of the body part; determine, based on the captured images, a location of a treatment object in the captured images; generate, based on the captured images, a three-dimensional representation of the surface of the body part, the three-dimensional representation including an indication of the treatment object in a location corresponding to the determined location of the treatment object in the captured images, and an indication of the location of the treatment element as determined by the localization unit; and provide, for presentation on the display to the user, the three-dimensional representation of the surface of the body part, including the indication of the treatment object and the indication of the location of the treatment element.

In some embodiments, the processor may be configured to generate, based on the captured images, a three-dimensional reference map of the surface of the body part; and generate a three-dimensional feature map by mapping the treatment object into the three-dimensional reference map. The processor may be configured to generate the three-dimensional representation of the surface of the body part by merging the three-dimensional feature map into the three-dimensional representation.

The at least one image capture device may, in some embodiments, comprise a three-dimensional depth camera and an imaging camera. The processor may be configured to generate a three-dimensional reference map of the surface of the body part based on images captured using the three-dimensional depth camera; determine the location of the treatment object in the images captured using the imaging camera; and generate a three-dimensional feature map by mapping the treatment object into the three-dimensional reference map. The processor may be configured to generate the three-dimensional representation of the surface of the body part by merging the three-dimensional feature map into the three-dimensional representation.

In some embodiments, the localization unit may comprise a further processor configured to determine the location of the treatment element of the treatment applicator relative to the body part based on at least one image captured by the at least one image capture device during the treatment.

The processor of the system may be configured to determine the location of the treatment object by processing the captured images to detect the treatment object in the captured images; and determining the location of the detected treatment object.

In some embodiments, the system may further comprise a user input device configured to receive from the user a selection of a type of treatment object to be treated. The processor may be configured to determine a location of a treatment object of the selected type in the captured images.

The system may further comprise a user input device configured to receive from the user a selection of display parameters regarding the presentation of the three-dimensional representation of the surface of the body part. The processor may be configured to provide the three-dimensional representation for presentation on the display to the user based on the selected display parameters.

In some embodiments, the processor may be configured to modify an appearance of the indication of the treatment object in the three-dimensional representation after treatment of the treatment object by the treatment applicator.

The at least one image capture device, the display and the processor may, in some embodiments, form part of a handheld electronic device.

In some embodiments, the localization unit may comprise at least one of: a device to determine the location of the treatment element of the treatment applicator relative to the body part from the captured images; and an inertial measurement unit.

The body part of the user may comprise the user's head, face or neck. The treatment object may comprise a treatment object on the skin of the user's head, face or neck.

According to a second specific aspect, there is provided a computer-implemented method of providing guidance to a user during treatment, using a treatment applicator, of a treatment object on the surface of a body part of the user. The method comprises receiving a plurality of images of the surface of the body part captured using at least one image capture device; determining, based on the captured images, a location of a treatment object in the captured images; receiving from a localization unit, an indication of a location of a treatment element of the treatment applicator relative to the body part; generating, based on the captured images, a three-dimensional representation of the surface of the body part, the three-dimensional representation including an indication of the treatment object in a location corresponding to the determined location of the treatment object in the captured images, and an indication of the location of the treatment element as received from the localization unit; and providing, for presentation to the user, the three-dimensional representation of the surface of the body part, including the indication of the treatment object and the indication of the location of the treatment element.

The method may further comprise generating, based on the captured images, a three-dimensional reference map of the surface of the body part; and generating a three-dimensional feature map by mapping the treatment object into the three-dimensional reference map. Generating the three-dimensional representation may comprise merging the three-dimensional feature map into the three-dimensional representation.

In some embodiments, the method may further comprise receiving, via a user interface, a user input comprising at least one of: a selection of a type of treatment object to be treated, the selection to be used to determine a location of a treatment object of the selected type in the captured images; a selection of display parameters regarding the presentation of the three-dimensional representation of the surface of the body part, the selection to be used by the processor to provide the three-dimensional representation for presentation to the user based on the selected display parameters; or a selection of a region of the user's body part to be the subject of the treatment, the selection to be used by the processor to provide for presentation on the display a treatment object appearing within the selected region.

According to a third specific aspect, there is provided a computer program product comprising a non-transitory computer-readable medium, the computer-readable medium having computer-readable code embodied therein, the computer-readable code being configured such that, on execution by a suitable computer or processor, the computer or processor is caused to perform steps of the methods disclosed herein.

These and other aspects will be apparent from and elucidated with reference to the embodiment(s) described hereinafter.

### BRIEF DESCRIPTION OF THE DRAWINGS

Exemplary embodiments will now be described, by way of example only, with reference to the following drawings, in which:
Fig. 1 is an illustration of an example of a treatment guidance system according to various embodiments;
Fig. 2 is an illustration of various examples of treatment applicators;
Fig. 3 is a schematic illustration of a further example of a treatment guidance system according to various embodiments;
Fig. 4 is a flowchart of an example of a method of providing guidance to the user during treatment according to various embodiments;
Fig. 5 is a flowchart of a further example of a method of providing guidance to the user during treatment according to various embodiments; and
Fig. 6 is a schematic illustration of an example of a processor in communication with a computer-readable medium.

### DETAILED DESCRIPTION OF THE EMBODIMENTS

Various examples of the invention disclosed herein provide a treatment guidance system that enables a user to perform a treatment activity in respect of part of their body more easily and with greater accuracy. More specifically, examples disclosed herein enable a user to position a treatment applicator accurately with respect to the part of their body being treated even if the treatment applicator itself or another part of their body (e.g. their hand) obscures the user's view of the body part, for example in their reflection in the mirror.

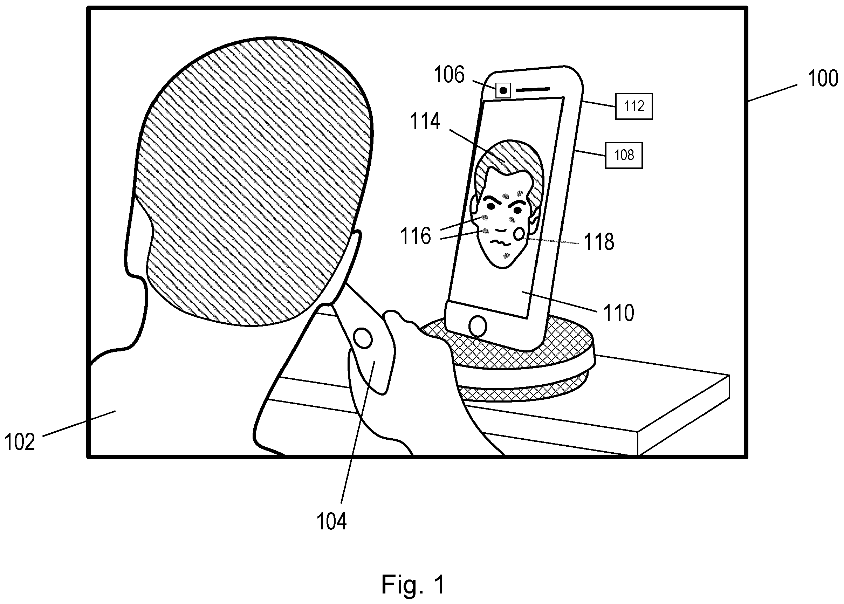

According to a first aspect of the invention, a system is provided. Referring to the drawings, Fig. 1 is an illustration of an example of a system 100, which may be referred to as a treatment guidance system. The treatment guidance system 100 is for guiding a user 102, using a treatment applicator 104, during the treatment of a treatment object on the surface of a body part of the user. As used herein, a "treatment object" may be considered to refer to any object on the surface of the user's body part that may be treated including, for example, a spot, a pimple, a lesion, a wrinkle, an area of dry skin, scar tissue, a wound or the like. In some examples, the object to be treated may be referred to as an "abnormality". The term "treatment" as used herein is intended to refer to an act performed specifically in respect of the treatment object intended to cause a change in its nature and/or its appearance. Thus, in some examples, the treatment of a treatment object may include the treatment of an abnormality, such as the application of laser energy to acne on a person's back or face while, in other examples, the treatment of a treatment object may include the application of make-up to an object to be covered on the skin on a person's face.

According to some embodiments of the present disclosure, the body part of the user 102 may comprise the user's head, face and/or neck. In such embodiments, the treatment object may comprise a treatment object on the skin of the user's head, face and/or neck. However, it will be appreciated that the system 100 may be used during the treatment of any treatment object on any body part of the user 102.

The system 100 comprises at least one image capture device 106, a localization unit 108, a display 110 and a processor 112. The processor 112 may be in communication with the image capture device 106, the localization unit 108 and/or the display 110. The at least one image capture device 106 is configured to capture an image or multiple images of the surface of the body part of the user 102. In the example shown in Fig. 1, a single image capture device 106 is shown, but it will be appreciated that, in other examples, multiple image capture devices may be included. The localization unit 108 is configured to determine a location of a treatment element (not shown in Fig. 1) of the treatment applicator 104. In the example shown in Fig. 1, the user 102 is using a treatment device as the treatment applicator 104. The treatment device shown in Fig. 1 may, for example, comprise a laser device used to apply a short laser pulse to a spot or to acne on a user's skin.

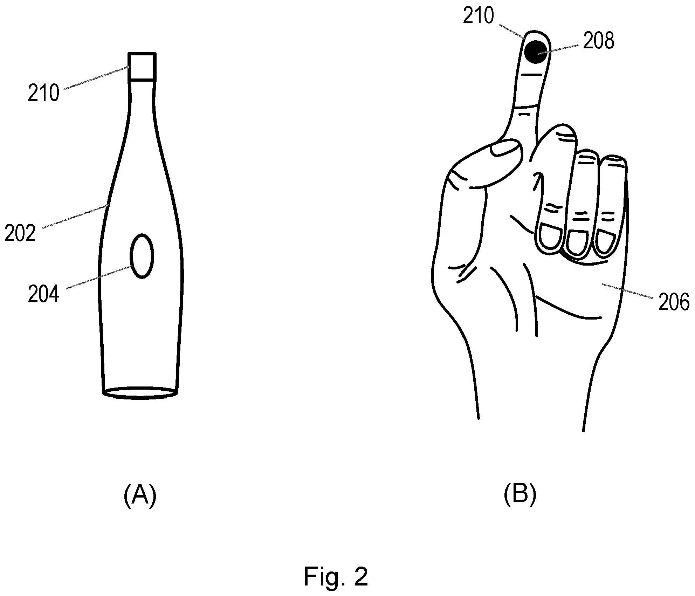

Examples of two different treatment applicators 104 shown in Fig. 2. In the example shown in Fig. 2A, a treatment device 202 is shown. The treatment device 202 may, for example, comprise the laser device shown in Fig. 1, or some other device configured to apply treatment to a treatment object. The treatment device 202 includes a treatment element 210 to apply the treatment to the treatment object on the surface of the body part of the user 102. In one example, the treatment element 210 may comprise the portion of the treatment device 202 from which the laser pulse is emitted. The treatment device 202 may further comprise a user control interface 204 to enable a user to cause a laser pulse to be emitted from the treatment element 210. In the example shown in Fig. 2B, the treatment applicator 104 comprises a hand 206 of the user 102. In this example, the treatment element 210 may be considered to comprise the tip of a finger of the user's hand 206, which may be used to apply a topical 208 (e.g. a cream or lotion) the treatment object on the surface of the body part of the user 102. Other types of treatment applicators 104 may alternatively be used.

Referring again to Fig. 1, the localization unit 108 may, in some embodiments, be implemented using the processor 112 while, in other embodiments, the localization unit may comprise a stand-alone component. For example, the localization unit 108 may comprise a software module configured to apply an algorithm or other processing technique to determine, from the captured images, the location of the treatment element 210 of the treatment applicator 104 relative to the body part of the user 102. For example, the processor 112 may determine the location of a defined reference point on the treatment element 210 relative to reference points (e.g. facial landmarks such as corners of the eyes, nose, mouth and/or ears) of the surface of the body part of the user 102. In other embodiments, the localization unit 108 may comprise a reference device (e.g. a device worn in a defined position by the user, such as a headband worn on the user's head) relative to which the location of the treatment element 210 of the treatment applicator 104 can be determined. In such examples, the treatment applicator 104 may comprise a component capable of communicating with the reference device and/or a component capable of recording motion data in respect of the treatment element, such as an inertial measurement unit (IMU). Thus, the localization unit 108 may comprise at least one of: a device to determine the location of the treatment element 210 of the treatment applicator 104 relative to the body part from the captured images; and an inertial measurement unit. The determined location of the treatment element 210 may be communicated to the processor 112.

The display 110 is configured to present information to the user 102 as discussed in greater detail below. In some examples, such as the example shown in Fig. 1, the at least one image capture device 106, the display 110 and the processor 112 may form part of a handheld electronic device. Such a handheld electronic device may, for example, comprise a smartphone, a tablet computer, a laptop computer, a desktop computer, an interactive mirror, or a wearable device, such as a smart watch. In the example shown in Fig. 1, the at least one image capture device 106 comprises a camera of a smartphone, the display 110 comprises the display screen of the smartphone and the processor 112 comprises the processing unit of the smartphone. Operation of the system 100 may be enabled using software, such as an application running on the smart phone. In some examples, the localization unit 108 may also form part of the handheld device. In other examples, however, each of the components of the system 100 may be implemented as individual components, though one or more of the components of the system 100 may interact and/or communicate with one another.

The processor 112 is configured to control the at least one image capture device 106 to capture a plurality of images of the surface of the body part. In some embodiments, multiple images of the surface of the body part may be captured by the image capture device 106, such that a three-dimensional representation of the surface of the body part can be constructed using the captured images. Such a three-dimensional representation may be generated using a plurality of images of the surface of the body part taken from different angles. For example, if the body part to be treated is the face of the user, then the user 102 may be prompted to look directly at the image capture device 106 so that an image of the user's face can be captured from the front. The user 102 may then be prompted to turn their head to one side, then to the other side, so that images may be captured of both sides of the user's head. Where the body part to be treated is not the user's face, then the user 102 may be prompted to present the appropriate body part to the image capture device 106, at various angles, so that the image capture device can capture the plurality of images. In some examples, the user 102 may operate the image capture device 106 to cause the image capture device to capture the images while, in other examples, the image capture device may be configured to capture images automatically when the user's body part is positioned appropriately.

The processor 112 is configured to determine, based on the captured images, a location of a treatment object in the captured images. As discussed previously, a treatment object may comprise an object, such as a defect, an object or an abnormality, occurring on the surface of the body part, that is to be treated using the treatment applicator 104. Different types of treatment objects may be located in the captured images using different techniques. Some treatment objects may be detected and/or located in the captured images using image processing techniques. Thus, the processor 112 may, in some embodiments, be configured to determine the location of a treatment object by processing the captured images to detect the treatment object in the captured images, and determining the location of the detected treatment object. In one example, the color of individual pixels in the captured images and/or the color difference between adjacent pixels in the captured images may be analyzed and used to identify a treatment object. For example, redness appearing on a person's skin may be indicative of a spot or a pimple. More particularly, the arrangement of pixels of a particular color may be indicative of a particular type of abnormality or treatment object. For example, a circular region of red pixels in an image may be indicative of a spot or a pimple. In other embodiments, other characteristics of the surface of the body part (e.g. the skin) may be used to determine the nature of the surface, and to identify a treatment object, such as an abnormality. The processor 112 may be configured to locate a single treatment object on the surface of the body part of the user 102, or to locate multiple treatment objects. For example, the processor 112 may be configured to analyze the captured images in order to locate all of the treatment objects. In other examples, the processor 112 may be configured to locate all treatment objects of a particular type, as discussed in greater detail below.

By employing image processing techniques to locate the treatment object in the captured images, it may be possible to detect and locate objects that are to be treated that are invisible or barely visible to the human eye. For example, a pimple in its early stage of growth might not be easily visible to the user 102, but the treatment object (i.e. the pimple) and the surrounding region may have particular characteristics detectable using image processing techniques, which enable it to be detected. Treatment of abnormalities at such an early stage can be beneficial in preventing development of the abnormalities.

The processor 112 is configured to generate, based on the captured images, a three-dimensional representation of the surface of the body part, the three-dimensional representation including an indication of the treatment object in a location corresponding to the determined location of the treatment object in the captured images, and an indication of the location of the treatment element as determined by the localization unit. As discussed above, the processor 112 may, in some embodiments, generate the three-dimensional representation of the surface of the body part using multiple images captured using the image capture device 106. The processor 112 may provide an indication in the three-dimensional representation of the location of any located treatment objects, at a corresponding location in the three-dimensional representation. For example, if it is determined from the captured images that a pimple exists at the end of the user's nose, then the pimple may be indicated at the end of the nose in the three-dimensional representation. The three-dimensional representation also includes an indication of the location of the treatment element 210 of the treatment applicator 104. Thus, the processor 112 may receive or obtain data from the localization unit 108 indicative of the location of the treatment element 210, and this location may be mapped into the three-dimensional representation along with the location(s) of the treatment object(s).

In some embodiments, the three-dimensional representation may be generated by forming a three-dimensional map of the surface of the body part of the user 102, adding the locations of the treatment objects onto the three-dimensional map, then merging the three-dimensional map that includes the treatment object locations with a three-dimensional image of the surface of the body part, created using the captured images. Thus, in such examples, the processor 112 may be configured to generate, based on the captured images, a three-dimensional reference map of the surface of the body part. The processor 112 may be further configured to generate a three-dimensional feature map by mapping the treatment object into the three-dimensional reference map. The processor 112 may be configured to generate the three-dimensional representation of the surface of the body part by merging the three-dimensional feature map into the three-dimensional representation.

While, in some embodiments, a single image capture device 106, such as a camera, may be used to capture images of the surface of the body part of the user 102, in other embodiments, as noted above, multiple image capture devices may be used. For example, in one embodiment, the at least one image capture device 106 may comprise a three-dimensional depth camera and an imaging camera. A three-dimensional depth camera, also referred to as a stereo camera or a three-dimensional camera, may for example comprise a time-of-flight (TOF) sensor capable of capturing depth information relating to the subject being imaged (e.g. the surface of the body part). In some embodiments, the three-dimensional depth camera may be used to determine the location of the treatment element 210 of the treatment applicator 104. Thus, the localization unit 108 may comprise, or make use of data from, the three-dimensional depth camera. The processor 112 may be configured to generate a three-dimensional reference map of the surface of the body part based on images captured using the three-dimensional depth camera. The imaging camera (e.g. a two-dimensional imaging camera) may comprise an RGB (i.e. red, green, blue) camera capable of capturing images of the surface of the body part, including the treatment object(s). The processor 112 may be configured to determine the location of the treatment object in the images captured using the imaging camera. In some embodiments, the processor 112 may be further configured to generate a three-dimensional feature map by mapping the treatment object into the three-dimensional reference map. The processor 112 may be configured to generate the three-dimensional representation of the surface of the body part by merging the three-dimensional feature map into the three-dimensional representation.

Once the three-dimensional representation has been generated, the processor 112 is configured to provide, for presentation on the display 110 to the user 102, the three-dimensional representation of the surface of the body part, including the indication of the treatment object and the indication of the location of the treatment element. In the example shown in Fig. 1, a three-dimensional representation 114 of the user 102 is displayed, along with indications 116 of multiple treatment objects (e.g. abnormalities), in this case shown as dots, and an indication 118 of the location of the treatment element 210, in this case shown as a circle.

In some examples, such as the example shown in Fig. 1, additional information may be presented along with the three-dimensional representation, for example to cause the three-dimensional representation to have a closer likeness to the user 102. In some examples, the processor 112 may be configured to display indications of the treatment objects using a first indicator (e.g. blue dots or circles) and may be configured to display the indication of the location of the treatment element using a second indicator (e.g. a red dot or circle). In this way, the user 102 can tell with relative ease where the treatment element 210 of the treatment applicator 104 is positioned relative to the object to be treated on the surface of the body part.

In some embodiments, the processor 112 may determine when treatment has been applied to a particular treatment object. For example, if a treatment device (e.g. the treatment device 202) is used to apply the treatment, then activation of the treatment device (e.g. the generation of a laser pulse) when the treatment device is positioned appropriately with respect to a treatment object may be recognized as an indication that treatment has been applied to the particular treatment object. Similarly, if the finger of the user's hand 206 is used to apply the treatment, then detection that the finger has touched the surface of the body part appropriately with respect to the treatment object may be recognized as an indication that the treatment has been applied to the particular treatment object. The processor 112 may, in some embodiments, be configured to modify an appearance of the indication of the treatment object in the three-dimensional representation after treatment of the treatment object by the treatment applicator 104. For example, once treatment has been applied to a particular treatment object, the indication of that treatment object may be removed from the three-dimensional representation, or changed to display a different color (e.g. green) to indicate that treatment has been applied. In this way, the user 102 is able to determine which treatment objects have been treated and which treatment objects are still to be treated, thereby reducing the risk of applying multiple treatments to the same treatment object.

In the examples discussed above, the processor 112 performs all of the disclosed processing steps. In other examples, however, the localization unit 108 may comprise a further processor (e.g. a second processor) configured to determine the location of the treatment element 210 of the treatment applicator 104 relative to the body part based on at least one image captured by the at least one image capture device 106 during the treatment. In this way, the processor of the localization unit 108 may function independently of the processor 112, and at a separate (e.g. later) time than the images of the surface of the body part are captured and used for determining the treatment object location. By utilizing multiple processors for the various processing tasks, use of the system may be made according to two phases. In a first phase, images of the surface of the body part may be captured and used to generate the three-dimensional representation, including the indication of the treatment object (or treatment objects). The three-dimensional representation may be stored (e.g. in a storage unit such as a memory) for presentation to the user 102 at some later time. A second phase may involve the real-time localization of the treatment element 210 of the treatment applicator 104, by determining (e.g. using the additional processor of the localization unit 108) the location of the treatment element during treatment. The indication of the location of the treatment element 210 may then be added to, or merged into, the three-dimensional representation, and provided for presentation to the user 102 in real time. During the second phase, only the location of the treatment element 210 is determined in real-time, so processing requirements are reduced compared to if the three-dimensional representation of the surface of the body part were to be generated and maintained in real-time.

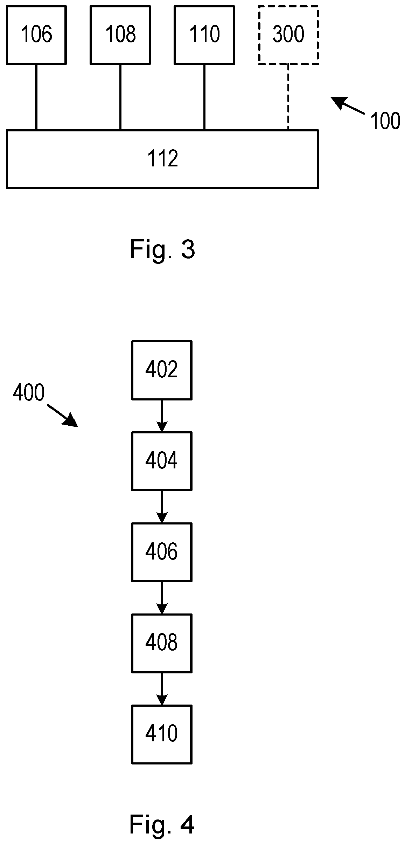

Fig. 3 is a schematic illustration of a further example of the system 100. In this example, the system 100 includes the at least one image capture device 106, the localization unit 108, the display 110 and the processor 112 in communication with the at least one image capture device, the localization unit and the display. In this example, each of the components is shown separately, though as mentioned above, two or more of the components of the system 100 may be combined or incorporated into a single device (e.g. a handheld electronic device).

The system 100 may further comprise a user input device 300, which may be referred to as a user interface. The user input device 300 may enable the user 102 to input information or data or to adjust parameters or settings of the system 100 according to personal preferences. In one example, the user input device 300 may enable the user 102 to provide a selection of a type of treatment object to be treated. For example, the user 102 may select a type or class of abnormality that they wish to treat, such as pimples, wrinkles, or the like. The processor 112 may be configured to determine a location of a treatment object of the selected type in the captured images. Thus, if the user 102 provides an input to the user input device 300 indicating that they intend to treat their pimples, then the processor 112 may detect and determine the location of one or more pimples in the captured images. The treatment object(s) of the selected type may then be displayed in the three-dimensional representation.

In another embodiment, the user input device 300 may enable the user 102 to provide a selection of display parameters regarding the presentation of the three-dimensional representation of the surface of the body part. For example, the user 102 may indicate: whether they would like indications of all treatment objects/abnormalities displayed in the three-dimensional representation, just those treatment objects/abnormalities meeting particular constraints (e.g. over a defined size) or just the treatment object currently being treated; whether they would like abnormalities to be displayed realistically or schematically (using a circle or a dot to represent a pimple); whether they would like the three-dimensional representation to be a realistic representation of the user 102 or of a generic person or an avatar; and/or whether they would like the three-dimensional representation to remain static on the display 110 or to move dynamically in response to a detected movement of the user 102 and, if the three-dimensional representation is to move dynamically, then whether it should represent a mirror image of the user.

A further aspect of the present invention relates to a method. Fig. 4 is a flowchart of an example of a method 400 which, in some embodiments, may comprise a computer-implemented method. The method 400 may be considered to be a method of providing guidance to a user 102 during treatment, in using a treatment applicator 104, of a treatment object on the surface of a body part of the user. The method 400 comprises, at step 402, receiving a plurality of images of the surface of the body part captured using at least one image capture device 106. In some embodiments, the method 400 may further comprise operating the at least one image capture device 106 to capture the plurality of images prior to receiving them. At step 404, the method 400 comprises determining, based on the captured images, a location of a treatment object in the captured images. As discussed herein, a treatment object may comprise any object on the surface of the body part that is intended to be treated, and may include, for example, an abnormality such as a pimple, a spot, a wrinkle or a lesion. The location of the treatment object may be determined relative to one or more objects or features (e.g. facial landmarks or other features of the body part) visible in the captured images.

The method 400 comprises, at step 406, receiving from a localization unit 108, an indication of a location of a treatment element 210 of the treatment applicator 104 relative to the body part. The localization unit 108 may, in some embodiments, determine the location of the treatment element 210 based on images captured in real-time showing the location of the localization unit relative to the body part. However, in other embodiments, the localization unit may determine the location of the treatment element 210 using some other technique. For example, as discussed above, and inertial measurement unit (IMU) may be used to determine the location of the treatment element 210. In some embodiments, a component in the treatment element 210 or in the treatment applicator 104 may be used to determine a location of the treatment element/applicator relative to a reference component positioned at a defined location (e.g. at a particular location on the user).

At step 408, the method 400 comprises generating, based on the captured images, a three-dimensional representation of the surface of the body part, the three-dimensional representation including an indication of the treatment object in a location corresponding to the determined location of the treatment object in the captured images, and an indication of the location of the treatment element as received from the localization unit 108. In some embodiments, the three-dimensional representation may be generated by merging a three-dimensional image of the body part (e.g. generated based on the captured images) with an indication of the treatment object (e.g. determined based on the captured images) and with an indication of the location of the treatment element 210 (determined using the localization unit 108). The method 400 comprises, at step 410, providing, for presentation to the user, the three-dimensional representation of the surface of the body part, including the indication of the treatment object and the indication of the location of the treatment element 210. The three-dimensional representation may, for example, be presented on the display 110.

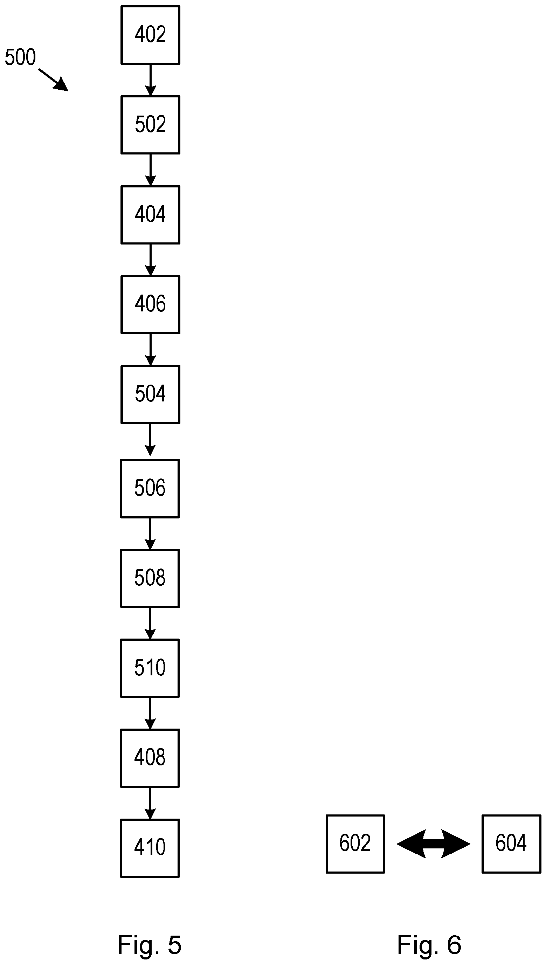

Fig. 5 is a flowchart of a further example of a method 500. Similar to the method 400 discussed above, the method 500 may comprise a computer-implemented method and may be considered to be a method of providing guidance to a user 102 during treatment, in using a treatment applicator 104, of a treatment object on the surface of a body part of the user. The method 500 may comprise one or more steps of the method 400 and/or steps corresponding to other functions performed by the processor 112 as disclosed herein.

The method 500 may further comprise. at various stages. receiving, via a user interface (e.g. the user input device 300) a user input. For example, the method 500 may comprise, at step 502, receiving, via a user interface, a selection of a type of treatment object to be treated, the selection to be used to determine a location of a treatment object of the selected type in the captured images. For example, the user 102 may indicate that they wish to locate pimples on their face, and in this case, the location of a pimple or multiple pimples may be determined in the captured images.

At step 504, the method 500 may comprise receiving, via a user interface, a selection of display parameters regarding the presentation of the three-dimensional representation of the surface of the body part, the selection to be used to provide the three-dimensional representation for presentation to the user 102 based on the selected display parameters. As discussed herein, the display parameters may define, among other things, the appearance of the surface of the body part (e.g. a likeness to the body part of the user 102), how the surface of the body part moves as the user moves, and/or the appearance of the treatment objects in the three-dimensional representation.

At step 506, the method 500 may comprise receiving, via a user interface, a selection of a region of the user's body part to be the subject of the treatment, the selection to be used to provide for presentation a treatment object appearing within the selected region. For example, the user 102 may select their forehead as the subject of the treatment, and a treatment object or multiple treatment objects located within the selected region (e.g. the forehead) will be included in the three-dimensional representation displayed to the user, without treatment objects located outside of the selected region.

The method 500 may comprise, at step 508, generating, based on the captured images, a three-dimensional reference map of the surface of the body part. The three-dimensional reference map may be generated using existing techniques. At step 510, the method 500 may comprise generating a three-dimensional feature map by mapping the treatment object into the three-dimensional reference map. Thus, the reference map of the surface of the body part (e.g. the user's face) is supplemented with indications of the locations of the treatment object or treatment objects. The step 408 of generating the three-dimensional representation may comprise merging the three-dimensional feature map into the three-dimensional representation. In other words, the three-dimensional image of the surface of the body part (e.g. the user's face) generated at step 408 is merged with the three-dimensional feature map which includes the location(s) of the treatment object(s).

One or more steps of the method 500, 600 may be performed using a processor or multiple processors, such as the processor 112 discussed above and/or the processor of the localization unit 108.

According to a further aspect of the invention, a computer program product is provided. Fig. 6 is a schematic illustration of an example of a processor 602 in communication with a computer-readable medium 604. According to various embodiments, a computer program product comprises a non-transitory computer-readable medium 604, the computer-readable medium having computer-readable code embodied therein, the computer-readable code being configured such that, on execution by a suitable computer or processor 602, the computer or processor is caused to perform steps of the methods 400, 500 disclosed herein.

The processor 112, 602 can comprise one or more processors, processing units, multicore processors or modules that are configured or programmed to control components of the system 100 in the manner described herein. In particular implementations, the processor 112, 602 can comprise a plurality of software and/or hardware modules that are each configured to perform, or are for performing, individual or multiple steps of the method described herein.

It will be apparent from this disclosure that embodiments disclosed herein provide a mechanism by which a user is able to perform treatment in respect of a treatment object on part of their body in an improved manner, by enabling the user to visualize the location of a treatment applicator being used relative to the treatment object. Thus, even if the user's hand or the treatment applicator were to obstruct the user's view of the treatment object, the user would be able to see the locations of the treatment object and the treatment applicator clearly relative to the body part as a result of the disclosed embodiments.

The term "module", as used herein is intended to include a hardware component, such as a processor or a component of a processor configured to perform a particular function, or a software component, such as a set of instruction data that has a particular function when executed by a processor.

It will be appreciated that the embodiments of the invention also apply to computer programs, particularly computer programs on or in a carrier, adapted to put the invention into practice. The program may be in the form of a source code, an object code, a code intermediate source and an object code such as in a partially compiled form, or in any other form suitable for use in the implementation of the method according to embodiments of the invention. It will also be appreciated that such a program may have many different architectural designs. For example, a program code implementing the functionality of the method or system according to the invention may be sub-divided into one or more sub-routines. Many different ways of distributing the functionality among these sub-routines will be apparent to the skilled person. The sub-routines may be stored together in one executable file to form a self-contained program. Such an executable file may comprise computer-executable instructions, for example, processor instructions and/or interpreter instructions (e.g. Java interpreter instructions). Alternatively, one or more or all of the sub-routines may be stored in at least one external library file and linked with a main program either statically or dynamically, e.g. at run-time. The main program contains at least one call to at least one of the sub-routines. The sub-routines may also comprise function calls to each other. An embodiment relating to a computer program product comprises computer-executable instructions corresponding to each processing stage of at least one of the methods set forth herein. These instructions may be sub-divided into sub-routines and/or stored in one or more files that may be linked statically or dynamically. Another embodiment relating to a computer program product comprises computer-executable instructions corresponding to each means of at least one of the systems and/or products set forth herein. These instructions may be sub-divided into sub-routines and/or stored in one or more files that may be linked statically or dynamically.

The carrier of a computer program may be any entity or device capable of carrying the program. For example, the carrier may include a data storage, such as a ROM, for example, a CD ROM or a semiconductor ROM, or a magnetic recording medium, for example, a hard disk. Furthermore, the carrier may be a transmissible carrier such as an electric or optical signal, which may be conveyed via electric or optical cable or by radio or other means. When the program is embodied in such a signal, the carrier may be constituted by such a cable or other device or means. Alternatively, the carrier may be an integrated circuit in which the program is embedded, the integrated circuit being adapted to perform, or used in the performance of, the relevant method.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A treatment guidance system (100) for guiding a user (102), using a treatment applicator (104), during treatment of a treatment object on a surface of a body part of the user, the system comprising:
at least one image capture device (106) configured to capture an image of the surface of the body part;
a localization unit (108) configured to determine a location of a treatment element of the treatment applicator relative to the body part;
a display (110) configured to present information to the user; and
a processor (112) configured to:
control the at least one image capture device to capture a plurality of images of the surface of the body part;
determine, based on the captured images, a location of a treatment object in the captured images;
generate, based on the captured images, a three-dimensional representation of the surface of the body part, the three-dimensional representation including an indication of the treatment object in a location corresponding to the determined location of the treatment object in the captured images, and an indication of the location of the treatment element as determined by the localization unit; and
provide, for presentation on the display to the user, the three-dimensional representation of the surface of the body part, including the indication of the treatment object and the indication of the location of the treatment element.

2. A treatment guidance system (100) according to claim 1, wherein the processor (112) is configured to:
generate, based on the captured images, a three-dimensional reference map of the surface of the body part; and
generate a three-dimensional feature map by mapping the treatment object into the three-dimensional reference map;
wherein the processor is configured to generate the three-dimensional representation of the surface of the body part by merging the three-dimensional feature map into the three-dimensional representation.

3. A treatment guidance system (100) according to claim 1, wherein the at least one image capture device (106) comprises a three-dimensional depth camera and an imaging camera; and
wherein the processor (112) is configured to:
generate a three-dimensional reference map of the surface of the body part based on images captured using the three-dimensional depth camera;
determine the location of the treatment object in the images captured using the imaging camera; and
generate a three-dimensional feature map by mapping the treatment object into the three-dimensional reference map;
wherein the processor is configured to generate the three-dimensional representation of the surface of the body part by merging the three-dimensional feature map into the three-dimensional representation.

4. A treatment guidance system (100) according to any of the preceding claims, wherein the localization unit (108) comprises a further processor configured to:
determine the location of the treatment element of the treatment applicator relative to the body part based on at least one image captured by the at least one image capture device during the treatment.

5. A treatment guidance system (100) according to any of claims 1 to 3, wherein the processor (112) is configured to determine the location of the treatment object by:
processing the captured images to detect the treatment object in the captured images; and
determining the location of the detected treatment object.

6. A treatment guidance system (100) according to any of the preceding claims, further comprising:
a user input device (300) configured to receive from the user a selection of a type of treatment object to be treated;
wherein the processor is configured to determine a location of a treatment object of the selected type in the captured images.

7. A treatment guidance system (100) according to any of the preceding claims, further comprising:
a user input device (300) configured to receive from the user a selection of display parameters regarding the presentation of the three-dimensional representation of the surface of the body part;
wherein the processor is configured to provide the three-dimensional representation for presentation on the display to the user based on the selected display parameters.

8. A treatment guidance system (100) according to any of the preceding claims, wherein the processor (112) is configured to:
modify an appearance of the indication of the treatment object in the three-dimensional representation after treatment of the treatment object by the treatment applicator.

9. A treatment guidance system (100) according to any of the preceding claims, wherein the at least one image capture device (106), the display (110) and the processor (112) form part of a handheld electronic device.

10. A treatment guidance system (100) according to any of the preceding claims, wherein the localization unit (108) comprises at least one of: a device to determine the location of the treatment element of the treatment applicator relative to the body part from the captured images; and an inertial measurement unit.

11. A treatment guidance system (100) according to any of the preceding claims, wherein the body part of the user comprises the user's head, face or neck, and wherein the treatment object comprises a treatment object on the skin of the user's head, face or neck.

12. A computer-implemented method (400) of providing guidance to a user during treatment, using a treatment applicator, of a treatment object on the surface of a body part of the user, the method comprising:
receiving (402) a plurality of images of the surface of the body part captured using at least one image capture device;
determining (404), based on the captured images, a location of a treatment object in the captured images;
receiving (406) from a localization unit, an indication of a location of a treatment element of the treatment applicator relative to the body part;
generating (408), based on the captured images, a three-dimensional representation of the surface of the body part, the three-dimensional representation including an indication of the treatment object in a location corresponding to the determined location of the treatment object in the captured images, and an indication of the location of the treatment element as received from the localization unit; and
providing (410), for presentation to the user, the three-dimensional representation of the surface of the body part, including the indication of the treatment object and the indication of the location of the treatment element.

13. A computer-implemented method (400, 500) according to claim 12, further comprising:
generating (508), based on the captured images, a three-dimensional reference map of the surface of the body part; and
generating (510) a three-dimensional feature map by mapping the treatment object into the three-dimensional reference map;
wherein generating (408) the three-dimensional representation comprises merging the three-dimensional feature map into the three-dimensional representation.

14. A computer-implemented method (400, 500) according to any of claims 12 to 13, further comprising:
receiving (502, 504, 506), via a user interface, a user input comprising at least one of:
a selection of a type of treatment object to be treated, the selection to be used to determine a location of a treatment object of the selected type in the captured images;
a selection of display parameters regarding the presentation of the three-dimensional representation of the surface of the body part, the selection to be used by the processor to provide the three-dimensional representation for presentation to the user based on the selected display parameters; or
a selection of a region of the user's body part to be the subject of the treatment, the selection to be used by the processor to provide for presentation on the display a treatment object appearing within the selected region.

15. A computer program product comprising a non-transitory computer-readable medium, the computer-readable medium (604) having computer-readable code embodied therein, the computer-readable code being configured such that, on execution by a suitable computer or processor (602), the computer or processor is caused to perform the method of any of claims 12 to 14.
